# EUROPÄISCHE PATENTSCHRIFT

(11) **EP 3 923 840 B1**
(45) Veröffentlichungstag und Bekanntmachung des Hinweises auf die Patenterteilung: **30.07.2025**
(21) Anmeldenummer: 20709466.5
(22) Anmeldetag: 24.01.2020
(51) Int. Cl.: A61B 17/70, A61B 17/86, A61B 90/00

(54) **KNOCHENVERANKERUNGSVORRICHTUNG FÜR DEN PEDIKELZUGANG**
BONE-ANCHORING DEVICE FOR A PEDICLE ACCESS
DISPOSITIF D'ANCRAGE OSSEUX POUR L'ABORD PÉDICULAIRE

(30) Priorität: 09.02.2019 DE 102019000965
(43) Veröffentlichungstag der Anmeldung: 22.12.2021
(73) Patentinhaber: Mimeo Medical GmbH, 70794 Filderstadt (DE)
(72) Erfinder: Schlenker, Heiter-Julian, 76185 Karlsruhe (DE)
(74) Vertreter: Stolmár & Partner Patentanwälte PartG mbB
(86) Internationale Anmeldenummer: PCT/DE2020/000008
(87) Internationale Veröffentlichungsnummer: WO 2020/160722

(56) Entgegenhaltungen:
- EP-A1- 2 740 428
- WO-A1-02/45606
- WO-A1-98/51241
- US-A1- 2010 331 895
- US-A1- 2012 143 266

## Beschreibung

Bei strukturell geschwächten Wirbelsäulen kann sich bei der Fixierung mit Knochenankern ein biomechanisches Problem einstellen, nämlich, dass der Knochen nicht lasttragend genug für eine ausreichende Verankerung mit Pedikelschrauben oder Ähnlichem bietet. Hierfür gilt die Augmentation mit Knochenzement des Wirbels als Stand der Technik. Eine funktionierende Alternative zu Pedikelschrauben mit Knochenzement ist dem aktuellen Stand der Technik nicht zu entnehmen.

Knochenzement basiert auf Polymethylmethacrylat (PMMA), wird während der OP aus zwei Komponenten zusammengemischt und steht für nur wenige Minuten zur Anwendung bereit. Bei der Anwendung und Injektion entstehen höhere Temperaturen während des Aushärtens, so dass Gefahr einer Überhitzung des Gewebes nicht ausgeschlossen werden kann. Dies kann eine Nekrosenbildung zur Folge haben. Des Weiteren besteht während der Injektion ein aktives Risiko eine lebensgefährliche Embolie auszulösen, z.B. sobald flüssiges PMMA in den Blutkreislauf gelangt und aushärtet. Auch das ungewollte Benetzen mit PMMA von anderen kritischen Strukturen (z.B. Dura oder Nervenwurzeln) ist möglich und deshalb Risiko behaftet. Einmal injiziert ist der ausgehärtete PMMA-Kunststoff nicht mehr aus dem Knochen entfernbar. Es bieten sich erschwerte Bedingungen bei einer Revision, so dass es schlussfolgernd gut wäre auf Knochenzement zu verzichten.

US5300074A zeigt ein Konzept zur Versorgung von Femurfrakturen, bei dem eine helikale Klinge zur rotationsstabilen Fixierung einer Femurkopf-Fraktur verwendet wird. WO9805263A1 zeigt ein ähnliches Konzept, wobei hier auf weitere biomechanische Vorteile eines klingelartigen Implantats eingegangen wird. Aus den Beschreibungen und den illustrierten Aufbauten beider Schriften ist ein weiterer biomechanischer Vorteil erkennbar. Die nach distal gerichtete und mit ca. 90° angewinkelte Platte im Femurkopf ist genau in die Lastrichtung rotiert, so dass maximal Knochenoberflächenkontakt in Lastrichtung generiert wird. Damit eignet sich diese Fixierungsmethode insbesondere für strukturschwächere Knochen.

Ein klingenartiger Knochenanker zur Stabilisierung von Wirbelsäulensegmenten wurde für den anterioren bzw. anterolateralen Zugang konzipiert (WO0245606A1). Der dort vorgestellte Knochenanker ist allerdings nicht für den posterioren Zugang durch einen Pedikelkanal geeignet. Die Anmelderin fordert für diesen Knochenanker eine spezielle Einbringorientierung, die sich nicht für die Implantation in den Pedikelkanal eignet. Es wird postuliert, dass die distale Klingenorientierung des Knochenankers parallel zur mediallateralen, bzw. distal-proximalen Ebene ausgerichtet ist und dann in den Wirbel implantiert. Diese Ausrichtung lässt allerdings keine Implantation in einen Pedikelkanal zu, da dieser eine andere Hauptausrichtung besitzt. Des Weiteren ist der dort vorgestellte Aufbau mehrteilig, was sich ungünstig seitens der Dauerfestigkeit auswirkt.

Die WO 98/52482 A1 offenbart eine Vorrichtung zur Fixation von Knochen, insbesondere von Wirbelkörpern relativ zueinander umfassend einen longitudinalen Längsträger 1 mit einer Zentralachse 2 und Verankerungselemente 3 mit Längsachsen 4, je einem vorderen Ende 5 und je einem hinteren Ende 6, wobei die Längsachsen 4 der Verankerungselemente 3 in einem Winkel zwischen 65° und 115° zur Zentralachse 2 des Längsträgers 1 stehen. Mindestens eines der Verankerungselemente 3 ist dabei klingenförmig ausgebildet, wobei das Verankerungselement 3 am hinteren Ende 6 Aufnahmemittel 7 für den Längsträger 1 mit Feststellmitteln 8, 13 zur lösbaren Fixierung der Verbindung zwischen Längsträger 1 und Verankerungselement 3 umfasst, und die fixierte Verbindung keine Relativbewegungen zwischen Längsträger 1 und Verankerungselement 3.

Die EP 2 740 428 A1 offenbart einen dynamischen Knochenanker, der ein Ankerelement mit einem röhrenförmigen Körper aufweist, welcher eine Ankerachse definiert. Das Ankerelement umfasst eine Vielzahl von Widerhakenelementen, wobei jedes Widerhakenelement zwischen einer ersten Position neben einer Oberfläche des röhrenförmigen Körpers und einer zweiten Position, die weiter von der Oberfläche des röhrenförmigen Körpers entfernt ist als die erste Position, beweglich ist. Zumindest ein Abschnitt einer freien Schneide der Widerhakenelemente ist in einem Winkel geneigt, der einem Spiralwinkel entspricht, der sich über zumindest eine volle Umdrehung um die Ankerachse erstreckt. Ein Längskernelement ist in dem röhrenförmigen Körper vorgesehen und weist einen ersten Abschnitt auf, der nicht mit dem Ankerelement verbunden ist, so dass das Ankerelement relativ dazu beweglich ist.

Die US 2012/143266 A1 offenbart eine polyaxiale Knochenschraubenanordnung, die einen Gewindeschaftkörper mit einem integrierten oberen Abschnitt umfasst, der in einer integrierten Aufnahmevorrichtung aufgenommen werden kann, wobei die Aufnahmevorrichtung einen oberen Kanal zur Aufnahme eines longitudinalen Verbindungselements und einen unteren Hohlraum aufweist, der mit einer unteren Öffnung zusammenwirkt. Ein Reibungspassungs-Kompressionseinsatz, ein planer, gespaltener Haltering und ein oberer Schaftabschnitt wirken zusammen, um eine Aufsteck- oder Einrastmontage des Schafts mit dem Empfänger entweder vor oder nach der Implantation des Schafts in einen Wirbel zu ermöglichen.

Die WO 98/51241 A1 offenbart eine Vorrichtung zum Implantieren von Bewehrungsstäben und ein Verfahren zu deren Verwendung. Die betreffenden Bewehrungsstahlvorrichtungen weisen eine ebene Oberfläche mit mindestens einer Ausstülpung auf, die ein im Wesentlichen kontinuierliches Querschnittsprofil besitzt. Der Krümmungsradius an jedem Punkt des Querschnittsprofils beträgt zwischen etwa 0,1 mm und 10 mm. Die betreffenden Bewehrungsstabvorrichtungen finden Verwendung in zahlreichen orthopädischen und verwandten Anwendungen, einschließlich der interfragmentären Fixierung, der Befestigung von Weichgewebe an Knochen oder der Befestigung von Platten oder Nägeln an Knochen und dergleichen.

Die US 2010/331895 A1 offenbart eine steosynthetische Vorrichtung zur Fixierung eines Knochens oder von Knochenfragmenten, der eine Längsachse hat und eine Knochenschraube mit einem Schaft umfasst, der ein Gewinde, ein vorderes Ende und ein hinteres Ende trägt, wobei das Gewinde einen maximalen Außendurchmesser aufweist. Ferner hat die Vorrichtung eine flügelartige Klinge, deren vorderes Ende mit dem vorderen Ende der Knochenschraube verbunden ist und deren hinteres Ende mit dem hinteren Ende der Knochenschraube verbunden ist. Die Klinge ist außerdem mit einer koaxialen Längsöffnung versehen, die eine Länge aufweist, die sich zwischen dem vorderen Ende und dem hinteren Ende erstreckt. Weiterhin ist die Klinge koaxial und drehbar auf dem Schaft der Knochenschraube montiert.

Die erfindungsgemäße Knochenverankerungsvorrichtung hat zur Aufgabe, eine Alternative zu zementaugmentierten Pedikelschrauben zu bieten, vor allem dann, sobald die innere knöcherne Struktur des Wirbels aufgrund einer Osteopenie oder sogar Osteoporose geschwächt ist. Des Weiteren soll durch die Art der Einbringung das Arbeiten mit Pedikelankern für den Chirurgen vereinfacht und beschleunigt werden.

Diese Aufgabe wird erfindungsgemäß von einer Knochenverankerungsvorrichtung, zum Verankern und Fixieren von Wirbeln, insbesondere zum Einführen in einen Pedikelkanal gemäß Anspruch 1 gelöst. Die Knochenverankerungsvorrichtung besitzt ferner, mit einem in einer Seitenansicht u-förmiges Aufnahmeelement für ein Korrekturelement, insbesondere einen Verbindungsstab, aufweisenden Gabelkopf mit zwei Schenkeln, die proximal auslaufen und einen Gewindeabschnitt formen, welcher im Eingriff mit einem Stellmittel steht, wobei die Schenkel einen radial äußeren Umfangsbereich aufweisen, in dem zum Ergreifen des Gabelkopfs mittels eines Handhabungsinstruments wenigstens eine Haltenut oder eine sonstige Instrumentenansetzstelle ausgebildet ist, und einem Knochenverankerungselement mit einen Kugelkopf, und das Knochenverankerungselement gegenüber dem Gabelkopf polyaxial verschwenkbar ist, und einem Druckstück, wobei das Druckstück distal das Knochenverankerungselement am Kugelkopf teilweise umgibt, und proximal einen Sitz für den Verbindungsstab formt, und das Knochenverankerungselement von distal kommend mit dem Gabelkopf und mit dem Druckstück montiert ist, dadurch gekennzeichnet, dass das Knochenverankerungselement einen hauptsächlich zylindrischen Kern besitzt, und der hauptsächlich zylindrische Kern am proximalen Bereich, sich wenigstens abschnittsweise von distal nach proximal im Durchmesser vergrößert, und zwei Flügel sich seitlich erstrecken, und die Flügel eine distale Flügelausrichtung und eine davon unterschiedliche proximale Flügelausrichtung (besitzen, und die Flügel sich helikal zwischen diesen Flügelausrichtungen ausformen und das Knochenverankerungselement nicht zum Einschrauben, sondern zum Einschlagen in den Knochen ausgebildet ist.

Die Struktur einer helikalen Anordnung von zwei Flügeln, die um einen hauptsächlich zylindrischen Kern angeordnet sind, bietet den Vorteil, dass das Knochenverankerungselement im Knochen rotationsstabil ist. Dies sind herkömmliche Pedikelschrauben nicht. Dass heiß mit dem erfindungsgemäßen Aufbau lassen sich auch korrektive Rotationsmomente in den Knochen einleiten, was mit bisher Pedikelschrauben nicht möglich war.

Wird das Knochenverankerungselement mit dem Gabelkopf mit Hilfe eines Stellmittels, wie beispielsweise eine Madenschraube, mit einem Verbindungsstab fixiert, wird ebenfalls die polyaxiale Verschwenkbarkeit des Gabelkopfs deaktiviert. Durch die Rotationsstabilität im Knochen und die ausgeschaltete Polyaxialität ist ein solches Knochenverankerungselement auszugsfest im Knochen. Denn eine Auszugsbewegung würde eine Drehung des Knochenverankerungselements erzwingen. Diese Drehung ist aufgrund der Verbindung zwei oder mehr Knochenverankerungsvorrichtungen entlang eines Verbindungsstabs nicht möglich. Zwei oder mehr Knochenverankerungsvorrichtungen entlang eines Verbindungsstabs halten den Verbindungsstab drehfest um die Verankerungspunkte und als Konsequenz bleibt das Knochenverankerungselement zugfest im Knochen sitzen.

Zur Steigerung der Auszugsfestigkeit im Knochen sind zusätzliche Zähne vorteilhaft. Vorzugsweise befinden sich diese Zähne im Pedikelbereich, damit sie sich mit dem Knochen bzw. mit der Pedikelinnenwandung verrasten können, um so eine optimale Effektivität gegenüber einer Auszugsbewegung am Knochenverankerungselements entgegenzuwirken. Zur Verrastung ist vorteilhaft, dass die Zähne auf mindestens einer elastischen Zunge arrangiert sind. Mit Hilfe der federelastischen Zunge können die Zähne während des Einbringens des Knochenverankerungselements ins Innere des Kerns ausweichen. Sie werden durch die knöcherne Struktur nach innen deformiert. Nachdem Einbringen des Knochenverankerungselements in den Pedikelkanal können mit Hilfe eines Hülsenelements die Zungen in die Ausgangsposition gebracht werden. Dies hat zur Folge, dass die Zähne in die Innenwandung des Pedikelkanals gedrückt werden. Das Knochenverankerungselement ist dann mit dem Knochen im Pedikelbereich verrastet. Weiterhin vorteilhaft ist es wenn, die Zähne seitlich angeordnet sind, d.h. nach medial und lateral gerichtet in den Pedikelkanal eingreifen. Nur dort haben sie Kontakt zur kortikalen Schicht des Pedikelkanals.

Zur weiteren Steigerung der Auszugsfestigkeit ist es vorteilhaft, wenn sich am Kern ein oder mehr umlaufende Rillen befinden und die Rillen in Umfangrichtung ein Profil aufweisen, welches haken- oder widerhakenähnlich ist. D.h. die Einführrichtung wird vereinfacht und eine Auszugsbewegung erschwert. Vorteilhaft ist es, wenn die umlaufenden Rillen nicht teilweise, sondern vollständig ausgeformt sind. Sie durchqueren die seitlichen Flügel und tragen zu einer Porosität der Flügel bei.

Eine Porosität der Flügel ist enorm von Vorteil für den Erfolg des Implantats, wenn es ohne zusätzlichen Knochenzement funktionieren soll. Knochenzellen wachsen verlangsamt und der dazu gehörige Stoffwechsel sind bei Vollmaterial-Implantaten unterbrochen. Deshalb ist eine poröse Struktur sehr von Vorteil. Vorzugsweise soll eine Porosität der Flügel gewählt werden, die dafür bekannt ist, dass Knochenzellen anwachsen und proliferieren. Das ist ein Bereich von 0,2mm bis 2,0mm, optimalerweise zwischen 0,4mm und 0,8mm.

Damit das erfindungsgemäße Knochenverankerungselement zu einer Funktionseinheit, d.h. Knochenverankerungsvorrichtung wird, muss es mit einem Gabelkopf montiert werden. Da die seitlichen Flügel eine gewissen Breite aufweisen, kann ein solches Knochenverankerungselement nicht von proximal in den Gabelkopf geführt und montiert werden. Das Knochenverankerungselement muss von distal in den Gabelkopf inseriert und montiert werden, damit die Knochenverankerungsvorrichtung als Implantat funktionstüchtig ist. Für diese Art von Gabelkopf-Montagen an Knochenankern von distal gibt es einen umfangreichen Stand der Technik. Als ein Beispiel hier dargestellt und nicht weiter ausdetailliert wird ein geschlitztes Druckstück gezeigt, welches von distal in den Gabelkopf geführt wird und anschließend kann der Kugelkopf des Knochenverankerungselements in das Druckstück von distal eingeklickt werden. Wird nun eine Kraft, z.B. durch das Anziehen eines Stellmittels (Madenschraube) und Verbindungsstabs erzeugt, wirkt diese Kraft auch auf das Druckstück. Durch eine äußere Konusfläche des Druckstücks und einer kongruenten Innenfläche des Gabelkopfs wird das Druckstück um den Kugelkopf gepresst. Eine Kraft, die durch das Stellmittel erzeugt wird, bewirkt die volle Verklemmung des Gabelkopfs und die Fixierung der Polyaxialität.

Das Knochenverankerungselement ist dadurch charakterisiert, dass es entlang des Kerns zwei seitliche Flügel besitzt. Diese Flügel besitzen einen helikalen Verlauf um die Kernmittelachse. Dabei wird eine Steigung zwischen 100mm bis 300mm vorgesehen, insbesondere 150mm bis 250mm, insbesondere 160mm bis 200mm. Optimalerweise besitzt das gesamt Sortiment an Knochenverankerungsvorrichtungen dieselbe Steigung, damit falls eine Knochenverankerungsvorrichtung mit anderer Länge oder anderen Durchmesser getauscht werden muss, derselbe vorpräparierte Kanal im Knochen verwendet werden kann. Über alle Längen des Knochenverankerungsvorrichtungs-Sets beträgt die Rotation der helikalen Außenflügel 60° bis 120°, insbesondere 70° bis 110°, insbesondere 80° bis 100°. Des Weiteren ist es von Vorteil, wenn ein Sortiment an Knochenverankerungsvorrichtungen mit unterschiedlichen Längen und Durchmesser denselben Formfaktor aufweisen.

Vorzugsweise ist das Knochenverankerungselement derart ausgebildet, dass es ermöglicht wird, die Knochenverankerungsvorrichtung zu implantieren, indem die Flügelausrichtung des distalen Endes des Knochenverankerungselements der Hauptausrichtung des Pedikelkanals entspricht. Dies entspricht nahezu einer kranialkaudalen Ausrichtung. Das Knochenverankerungselement wird durch Schläge in den Pedikelkanal eingetrieben. Dabei dreht sich das Knochenverankerungselement um die Zentralachse gemäß der zuvor definierten Steigung. In der finalen Position besitzt die distale Flügelausrichtung eine lateral-mediale Ausrichtung, wobei die proximale Flügelausrichtung die der Pedikelhauptausrichtung entspricht.

Nach der Implantation befindet sich das Knochenverankerungselement im Wirbel. Das Knochenverankerungselement ist ferner derart ausgebildet, dass sich die Flügel-Außenflächen am proximalen Bereich an den kranialen und kaudalen Bereichen des Pedikelkanals abstützen, oder sie zeigen in diese Richtungen. Distal stützt sich die projizierte Fläche, die sich durch den Kern und die seitlichen Flügelflächen ergibt, in der Spongiosa des Wirbels nach kranial/kaudal ab. Damit wird eine Verkippung der Knochenverankerungsvorrichtung unter Einleitung einer Flexion/Extensions-bewegung vermieden. Die Knochenverankerungsvorrichtung stützt sich optimal an den knöchernen Strukturen ab, oder zeigt zumindest in diese Richtung, und verteilt die resultierende Last homogener und breitflächiger als eine Pedikelschraube an das Spongiosa-Gewebe. Ebenso stützt sich die Knochenverankerungsvorrichtung proximal im hauptsächlich oval ausgeformten Pedikelkanal kranial und kaudal ab, oder sie zeigt mit den Flügelaussenkanten in diese Richtung. Sonstige Pedikelschrauben befinden sich als zylindrisches Objekt in einem ovalen Tunnel (=Pedikelkanal) nicht im biomechanischen Optimum.

Damit die Flügel-Außenflächen sich am proximalen Bereich nicht in die kaudalen und kranialen Bereiche des Pedikelbereich eindrücken oder sogar einschneiden, ist es vorteilhaft, wenn die Außenflächen der Flügel konvexe Krümmungen besitzen, um die Kontaktspannungen zu den kranialen und kaudalen Pedikelbereichen zu reduzieren.

Für eine bessere Lastverteilung der Biegemoment entlang des Knochenverankerungselements ist es vorteilhaft, dass die beiden Flügel sich am proximalen Bereich zum Kugelkopf hin verjüngen und auf der Außenkontur des Kerns enden. Des Weiteren kann der Kern am proximalen Bereich, genauer beim Halsbereich, abschnittsweise konisch verlaufen. Auch dies verteilt die Biegemomente und -spannungen besser im belasteten Bauteil.

Naturgemäß besitzen die Pedikelkanäle einen gewissen Formfaktor, welcher das Oval beschreibt. Dieser Formfaktor definiert den Zusammenhang aus Höhe und Breite. Optimalerweise sind die erfindungsgemäßen Knochenverankerungselementen genau daran angepasst, damit sie den ovalen Querschnitt am besten wiedergeben. Dabei weisen, die Knochenverankerungselemente, mit den beiden Flügeln eine Höhe (H), die zwischen den Außenkanten der Flügel definiert ist, und einen Außendurchmesser (D) des Kerns (inkl. Zähne), einen Formfaktor mit dem Verhältnis aus H/D auf, welcher zwischen 1,3 bis 2,5, vorzugsweise 1,4 bis 2,2, vorzugsweise 1,6 bis 2,0 beträgt.

Vorteilhaft für eine zusätzliche Steigung der Festigkeit und als letztes klinisches Mittel, falls die Knochenqualität zu niedrig ist, ist eine Kanülierung mit seitlichen Öffnungen vorgesehen. Durch diese kann Knochenzement injiziert werden. Vorteilhaft ist hier, dass die Orientierung der seitlichen Öffnungen nach der Implantation immer nach kranial und kaudal zeigen, wohin die größte Last innerhalb der Spongiosa gerichtet ist. Des Weiteren erweist es sich als vorteilhaft, wenn die Kanülierung unterschiedliche Durchmesser besitzt. Hier kann beispielsweise von proximal ein Hülsenelement eingesetzt werden.

Da es sich um eine relativ komplexe geometrische Struktur bei dem erfindungsgemäßen Knochenverankerungselement, als Teil der Knochenverankerungsvorrichtung handelt, ist es von Vorteil, wenn das Knochenverankerungselement einstückig mit Hilfe eines generativen Fertigungsverfahrens hergestellt wird. Hierzu zählen alle bekannten Verfahren des 3D-Drucks, wie z.B. das Laserstrahl-Schmelzen, Elektronenstrahl-Schmelzen, oder anderen additiven Verfahren. Geeignete Materialien sind alle implantierbare Materialien wie zum Beispiel: wie Titan-, CoCr- oder Edelstahl-Legierungen, oder Kunststoffen wie PEEK, PSU, PPSU, PEAK, PEK, faserverstärktes CFR-PEEK usw. Des Weiteren kann es von Vorteil sein, wenn geometrische Strukturen mit enger Toleranzvorgabe, wie z.B. der Kugelkopf, nachträglich mit einem abtragenden Verfahren (z.B. CNC-Drehen, CNC-Fräsen oder Erodieren) überarbeitet werden.

Weitere Merkmale und Einzelheiten der Erfindung ergeben sich aus den Patentansprüchen, den nachfolgenden Abbildungen und der nachfolgenden Beschreibung der dargestellten Ausführungsformen der erfindungsgemäßen Knochenverankerungsvorrichtung:
Fig. 1 zeigt die erfindungsgemäße Knochenverankerungsvorrichtung im Zusammenbau und in einer Explosionsdarstellung.
Fig. 2 zeigt die erfindungsgemäße Knochenverankerungsvorrichtung montiert mit einem Verbindungsstab und einem Stellmittel. Dies im Zusammenbau und als Explosionsdarstellung.
Fig. 3 illustriert zwei implantierte Knochenverankerungsvorrichtungen in einen Wirbel.
Fig. 4a zeigt die Knochenverankerungsvorrichtung vor dem Eintritt in den Pedikelkanal.
Fig. 4b zeigt die Knochenverankerungsvorrichtung nach der Implantation in der finalen Position.
Fig. 4c zeigt einen Querschnitt durch einen Pedikelkanal mit implantierter Knochenverankerungsvorrichtung.
Fig. 5 stellt den Wirbel mit zwei implantierten Knochenverankerungsvorrichtungen in drei Ansichten dar.
Fig. 6 vergleicht einen herkömmlichen Schraubenschaft einer Knochenschraube, wie er bei einer Pedikelschraube zum Einsatz kommt, mit dem erfindungsgemäßen Knochenverankerungselement.
Fig. 7 zeigt die dazugehörigen projizierten Flächen, die die Lastverteilung auf das Knochengewebe bewirken.
Fig. 8 unterschiedliche Schnitte durch die Knochenverankerungsvorrichtung.
Fig. 9a und Fig. 9b bilden das erfindungsgemäße Knochenverankerungselement ab, welches Zähne zur Verrastung im Pedikelkanal besitzt und das Hülsenelement, welches die elastische Zunge mit den Zähnen in Richtung Pedikelinnenwand drückt.
Fig. 10a und b zeigen jeweils ein Knochenverankerungselement, wobei die elastische Zunge einmal nach proximal und das andere Mal nach distal zeigt.
Fig. 11a und 11b zeigen ein Knochenverankerungselement mit einem drehbar gelagerten Knochengewindeabschnitt im Pedikelbereich.
Fig. 12a und b stellen ebenfalls eine Variante vor, die ein drehbar gelagerten Knochengewindeabschnitt besitzt.
Fig. 13 zeigt ein Knochenverankerungselement bei dem die äußeren Flächen der Flügel konvexe Krümmungen aufweisen, damit an der kranialen und kaudalen Pedikelinnenwand keine Spannungskonzentrationen entstehen.
Fig. 14 illustriert ein Knochenverankerungselement, bei dem die gegenüberliegenden Flügelflächen nicht parallel, sondern zum Kern hin verdickend verlaufen. Dies hat einen positiven Einfluss auf die Biegestabilität der Flügel.

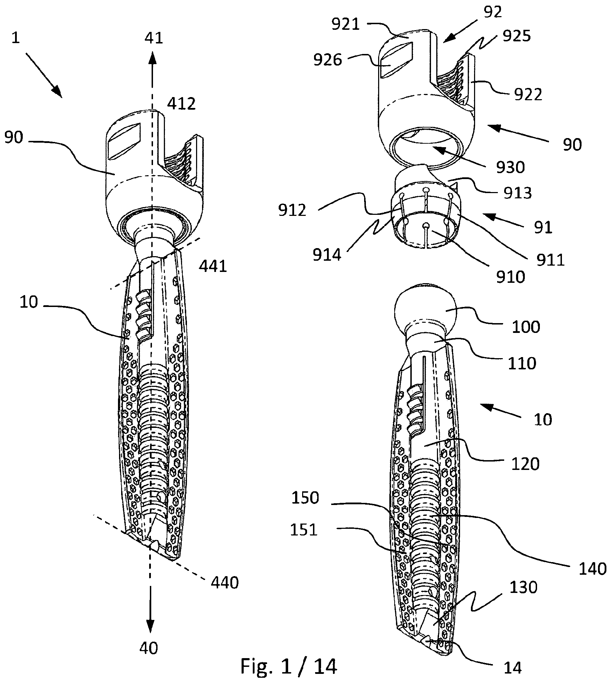

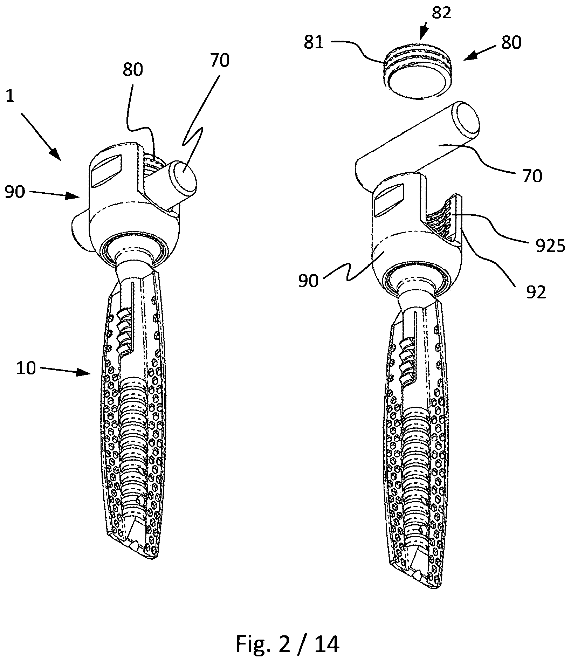

Fig. 1 und 2 zeigen die erfindungsgemäße Knochenverankerungsvorrichtung (1) bestehend aus einem Gabelkopf (90), einem Knochenverankerungselement (10) und einem Druckstück (91). Das Knochenverankerungselement (10) definiert eine Mittelachse entlang der Länge (412), welche sich von distal (40) nach proximal (41) erstreckt. Das Knochenverankerungselement (10) besitzt von proximal (41) nach distal (40) einen Kugelkopf (100), einen Halsbereich (110), einen Pedikelbereich (120), Kern (140) und einen distalen Bereich (130). Entlang vom zentralen und hauptsächlich zylindrischen Kern (140) sind zwei Flügel seitlich angeordnet (150, 151). Die Flügel besitzen proximal (441) und distal (440) jeweils eine Flügelausrichtung, die unterschiedlich voneinander ist. Entlang dieser beiden Flügelausrichtungen (440 und 441) formen sich die Flügel Helix-artig gemäß einer größeren Steigung. Details der Steigung sind zuvor offenbart. Gehalten wird das Knochenverankerungselement (10) durch ein Druckstück (91), welches den Kugelkopf (100) teilweise umschließt und in einen Sitz (910) aufnimmt. Dieser Sitz (910) ist elastisch verformbar, indem Federarme (911) durch entsprechende Schlitze (912) ausgebildet sind. Außen besitzt das Druckstück eine konische Fläche (914) die mit der Öffnung (930) des Gabelkopfs (92) in Kontakt steht. Final montiert befindet sich das Druckstück (91) im Gabelkopf (90). Das Druckstück (91) besitzt proximal einen Sitz (913) für einen Verbindungsstab (70). Dieser Sitz (913) ist derart gegenüber dem Gabelkopf (90) ausgerichtet, dass der u-förmige Ausschnitt (92) diesem entspricht. Der Gabelkopf (90) besitzt am proximalen Bereich zwei auslaufende Schenkel (921 und 922) die zusammen einen Gewindeabschnitt (925) formen, in dem ein Stellmittel (80), mit einem kongruenten Gewindebereich (81), eingeschraubt werden kann. Dafür besitzt das Stellmittel (80) einen Werkzeuganschluss (82), welcher nicht gezeigt wird. Für den Werkzeuganschluss (82) kommen Torx, Vielzahnrund, Sechskant, Vierkant-anschlüsse u.a. in Frage. Der Gabelkopf (90) besitzt am äußeren proximalen Umfang Vorrichtungen (926), wie beispielsweise eine Haltenut oder Vertiefungen, die dazu geeignet sind ein Instrument daran zu befestigen.

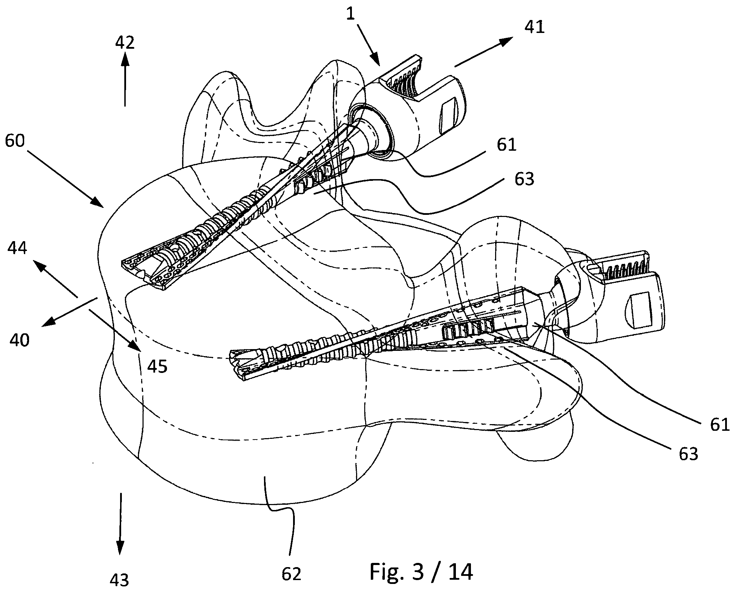

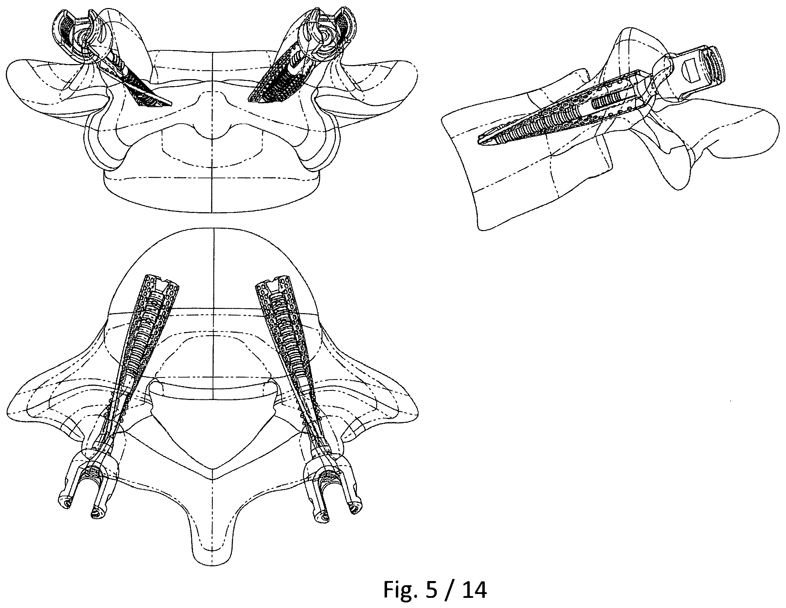

Fig. 3 und 5 zeigen in einen Wirbel (60) eine paarweise angeordnete Implantation von Knochenverankerungsvorrichtungen (1). Dabei lassen sich verschiedene anatomische Richtungen definieren. Distal (40) und proximal (41) ergeben sich aus der zuvor erwähnten Definition. Davon seitlich sind die laterale (44) und mediale (45) Richtung und davon senkrecht die kraniale (42) und kaudale (43) Richtung definiert. Der Wirbel (60) lässt sich in für die erfindungsgemäße Knochenverankerungsvorrichtung (1) wichtige Bereiche einteilen; Pedikeleintritt (61), Pedikelkanal (63) und der innenliegenden Spongiosa (62).

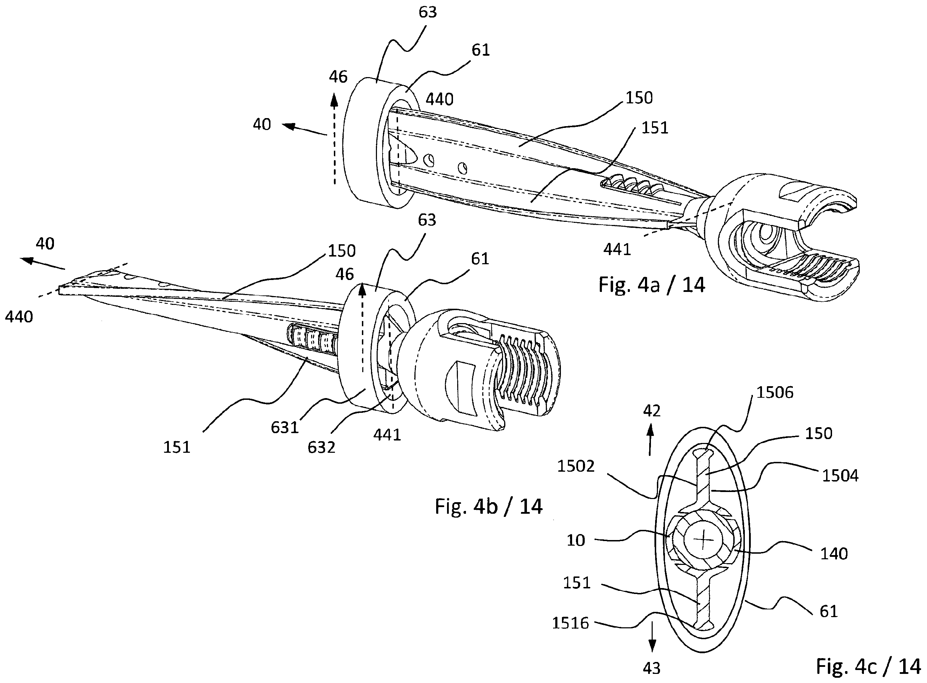

In Fig. 4a, b und c ist der Vorgang der Implantation in den Pedikelkanal (63) dargestellt. Der Pedikelkanal (63) besitzt eine Kortikalis (631) und einen inneren spongiösen Bereich (632). In Fig. 4a ist erkennbar, dass das distale Ende des Knochenverankerungselements (10) in den Pedikeleintritt (61) geführt wird. Dabei besitzt der Pedikelkanal im Schnitt (Fig. 4c) eine ovale Form, wobei die längere Erstreckung des Ovals in kranial-kaudaler Richtung (42, 43) orientiert ist. Dies entspricht der Pedikelausrichtung (46). Beim Einführen des Knochenverankerungselements (10) in den Pedikeleintritt (61) muss der Chirurg darauf achten, dass die distale Flügelausrichtung (440) der Pedikelausrichtung (46) entspricht. Nachdem das Knochenverankerungselement (10) in den Wirbel (60) eingeschlagen wurde (Fig. 4b), entspricht nun die proximale Flügelausrichtung (441) der Pedikelausrichtung (46). D.h. im Schnitt betrachtet bleibt das Profil des Knochenverankerungselements (10) identisch im Pedikelkanal (Fig. 4c). Das Knochenverankerungselement (10) verschiebt sich während der Implantation in das Wirbelinnere (62) entlang der vorgegebenen Helix, die durch die Flügel (150, 151) erzwungen wird. Im fertig implantierten Zustand befindet sich die distale Flügelausrichtung (440) in einer seitlichen Ausrichtung, d.h. die Flügel (150, 151) zeigen nach lateral und medial. In Fig. 4c ist weiterhin gezeigt, dass das Knochenverankerungselement (10) dem ovalen Bereich des Pedikels (63) recht gut approximiert. Kranial (1506) und kaudal (1516) besitzt das Knochenverankerungselement (10) vorzugsweise abgerundete Außenflächen, die ein Einschneiden, Eindrücken oder Spalten der kortikalen Schicht des Pedikels verhindern. Dabei können die Außenflächen (1506, 1516) über die seitlichen Wandungen der Flügel (1502, 1504 und 1512, 1514) hinüberragen.

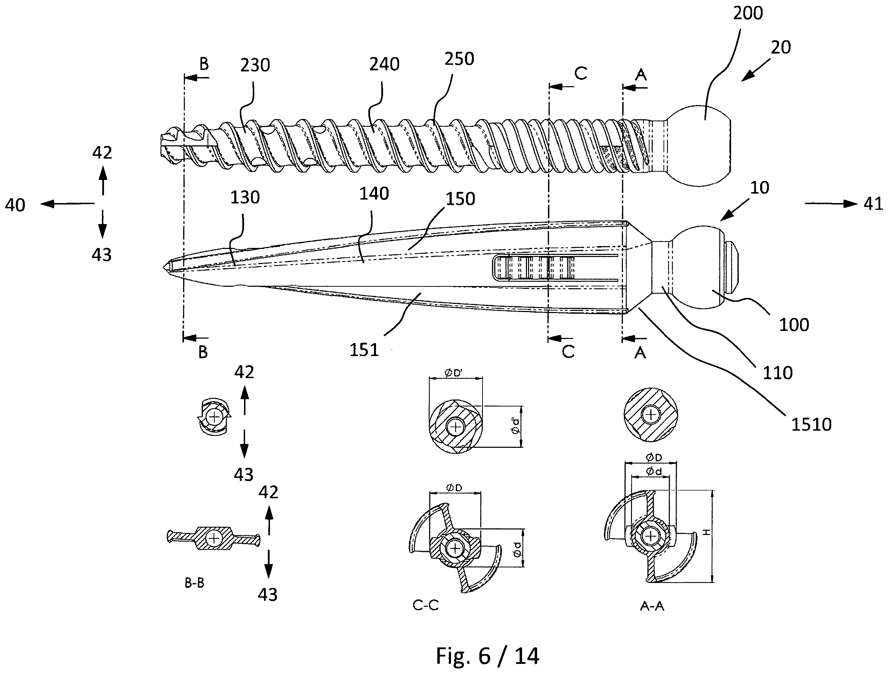

Fig. 6 zeigt einen direkten Vergleich aus einer herkömmlichen Knochenschraube (20) und dem Knochenverankerungselement (10) der erfindungsgemäßen Knochenverankerungsvorrichtung (1). Strukturell ähnlich sind der Kugelkopf (100, 200) und Halsbereich (110, 210). Beide Knochenanker (10 und 20) besitzen einen Kern (140 und 240) und einen distalen Bereich (130 und 230). Bei der Knochenschraube (20) ist ein Knochengewinde (250) vorgesehen, was gleichzeitig den Außendurchmesser (D') der Knochenschraube definiert. Der Kern (240) entspricht dem Innendurchmesser (d'). Analog hierzu kann am Knochenverankerungselement (10) der Außendurchmesser (D) anhand des äußeren Umfangs der Zähne (122) verortet werden. Des Weiteren kann der Kerndurchmesser (d) durch den Kern (140) selbst definiert werden. Damit entsprechen die Durchmesser D'=D als auch d'=d für einen direkten Vergleich. Als deutlich erkennbarer Unterschied zur Knochenschraube sind beim Knochenverankerungselement (10) zwei Flügel, ein erster Flügel (150) und ein zweiter Flügel (151) vorgesehen. Sie definieren eine Breite (H). Das Verhältnis aus der Breite (H) zum Durchmesser (D) wird als Formfaktor definiert. Die vorzugsweisen Bereiche des Formfaktors wurden bereits erwähnt. Vorteilhaft ist es, wenn die Breite der beiden Flügel (150, 151) zum Kugelkopf (100) hin sich graduell verkleinert (1510) und im Kern (140) mündet. Dies hat einen positiven Effekt auf den Biegespannungsverlauf.

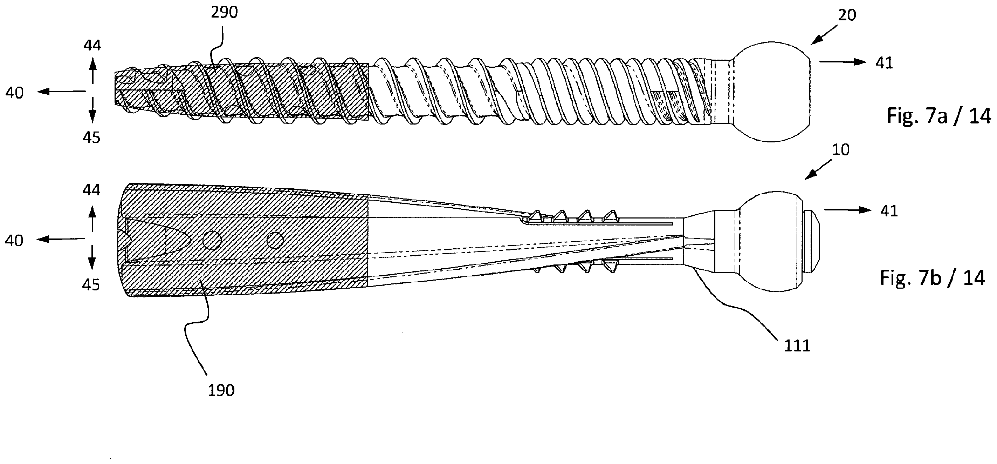

Fig. 7a und 7b illustrieren den direkten Vergleich der lasttragenden projizierten Flächen (190, 290) in der Spongiosa (62) eines Wirbels (60). Fig. 7a zeigt die projizierte Fläche (290) einer Knochenschraube (20), die bei einer Flexion/Extensionsbewegung innerhalb der Spongiosa belastet wird. Fig. 7b zeigt die projizierte Fläche des erfindungsgemäßen Knochenverankerungselements (10). Eindeutig zu sehen ist, dass diese Fläche (190) deutlich größer ist als die Fläche einer Knochenschraube (290). Je größer diese projizierte Fläche, desto mehr Last kann auf ein weiches Spongiosa-Gewebe übertragen werden. Insgesamt kann, je nach Formfaktor, fast von einer Verdopplung dieser aktivlasttragenden Fläche ausgegangen werden. In dieser Aufnahmerichtung kann ergänzend der vorteilhafte Verlauf einer Durchmesservergrößerung (111) zwischen und/oder beim Pedikelbereich (120) und dem Halsbereich (110) identifiziert werden. So wird sichergestellt, dass der Halsbereich eine ausreichende Biegesteifigkeit aufweist. Die Durchmesservergrößerung kann konisch, abschnittsweise konisch oder anhand von Rundungen verlaufen.

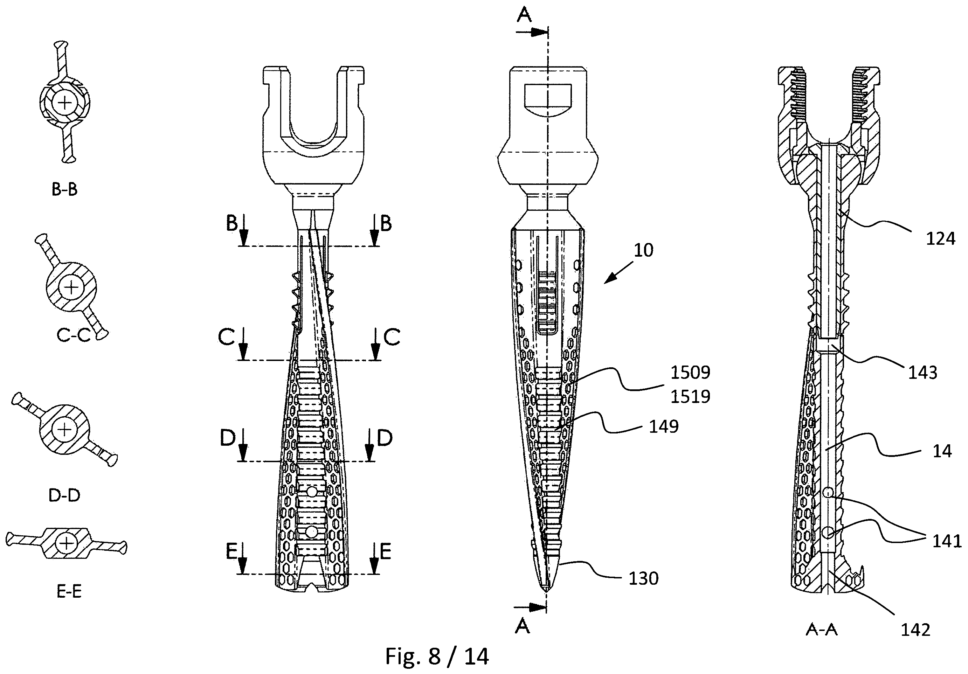

Fig. 8 zeigt die erfindungsgemäße Knochenverankerungsvorrichtung (1) in zwei Ansichten sowie einige Schnitte orthogonal der Mittelachse (412). Zu erkennen ist, dass die Flügel (150, 151) eine unterschiedliche Ausrichtung aufweisen. Im Longitudinalschnitt ist die Kanülierung (14) mit den seitlichen Fenestrationsöffnungen (141) zu erkennen. Vorzugsweise besitzt die Kanülierung unterschiedliche Durchmesser (142 ,143). Einerseits soll so ein Fluid-Widerstand am distalen Ende der Kanülierung erhöht werden, indem der Durchmesser nach distal reduziert wird (142). Andererseits ist es von Vorteil, wenn die Kanülierung (14) gleichzeitig als Sitz für ein Hülsenelement (124) verwendet werden kann. Hierfür kann eine Durchmesservergrößerung (143) vorgesehen werden. In einer Ansicht der Knochenverankerungsvorrichtung (1) ist zu erkennen, dass die Flügel (150, 151) durch eine Porosität bzw. durch mehrere Öffnungen (1509, 1519) charakterisiert sind. Sie dienen dem Anwachsen und der Integration von Knochenzellen. Eine bevorzugte Porengröße wurde bereits erwähnt. Des Weiteren ist es von Vorteil, wenn der Kern (140) ein oder mehr umlaufende Rillen (149) besitzt. Diese Rillen weisen ein im Profil haken-ähnliches Profil auf. Dies verbessert die Auszugsfestigkeit des Knochenverankerungselements (10) im Knochen.

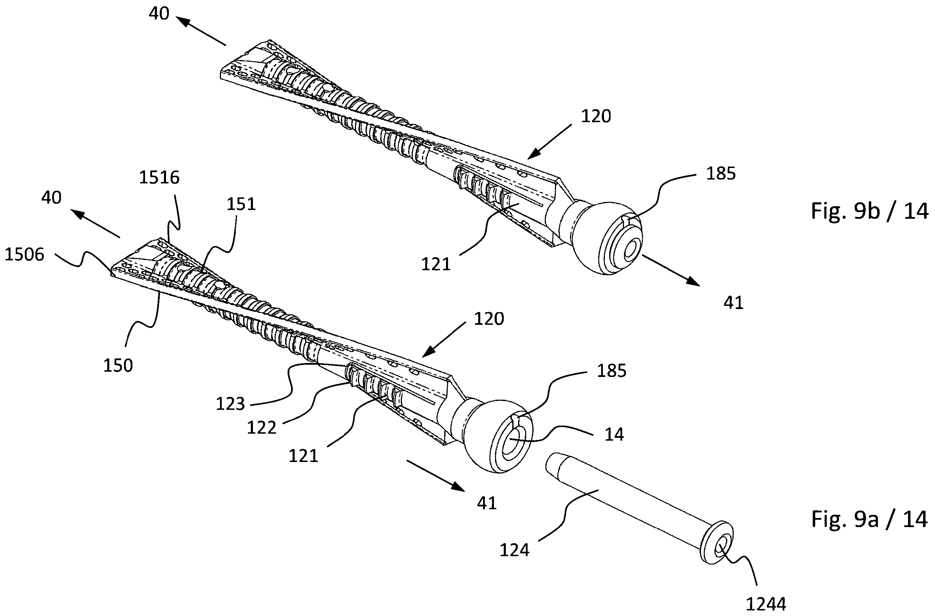

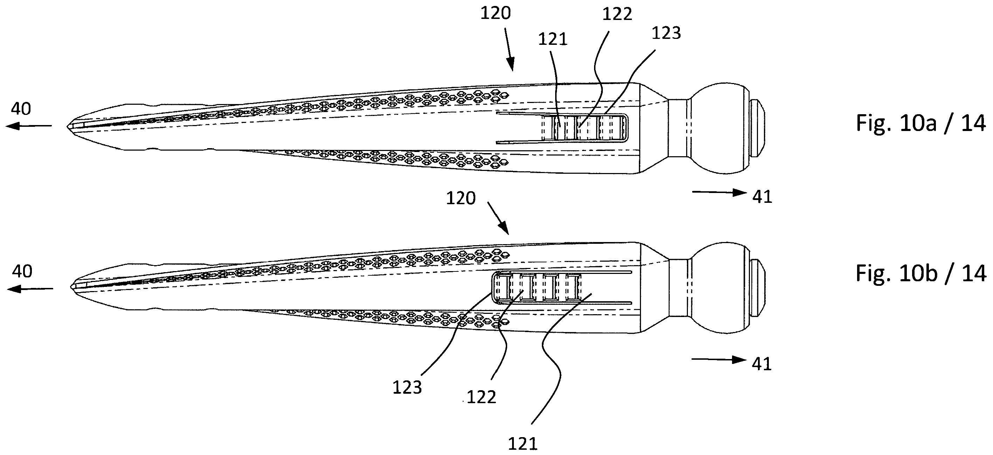

Fig. 9a und 9b zeigen das Knochenverankerungselement (10) in Verwendung eines Hülsenelements (124), welches in die Kanülierungsöffnung (14) geführt werden kann. Fig. 9a zeigt den Zustand bei den die elastischen Federarme (121) mit den darauf befindlichen Zähnen (122) ins Kerninnere einfedern können. Die elastischen Federarme (121) werden durch einen oder mehr Schlitze (123) gebildet. Hier dargestellt sind u-förmige Schlitze. Nachdem das Hülsenelement (124) in der Kanülierung (14) eingeführt ist (Fig. 9b), können die elastischen Federarme (121) nicht mehr einfedern. Sie werden durch den Hülsenelementkörper (124) gehindert. Des Weiteren wird durch das Einstecken des Hülsenelements (124) eine aktive Verdrängung der elastischen Federarme (121) aus dem Kanülierungsbereich erwirkt. Somit ist es mit Hilfe der Einführung des Hülsenelements möglich, dass nach der Implantation des Knochenverankerungselements (10) die Zähne (122) in die Pedikelwandung gedrückt werden können. Idealerweise befinden sich die Zähne (122) auf Höhe des Pedikelbereichs (120). In diesem Bereich (120) können sie die bestmögliche Wirkung der Verrastung mit dem Pedikelkanal (63) erzielen. Fig. 10a und 10b zeigen dabei eine unterschiedliche Richtung, in die elastischen Federarme angeordnet sein können. Ebenfalls in Fig. 9a und 9b dargestellt ist ein Indikator (185), welcher dazu dient die distale Flügelausrichtung (440) und/oder proximale Flügelausrichtung (441) dem Anwender anzuzeigen. Als Indikator, können asymmetrische Ausformungen, Schlitze, Auftragungen oder auch Beschriftungen dienlich sein. Des Weiteren ist es von Vorteil, wenn der Indikator gleichzeitig als Schnittstelle für ein chirurgisches Instrument dient und dadurch die Orientierung der Flügelausrichtungen (440, 441) abseits des Implantats anzeigt.

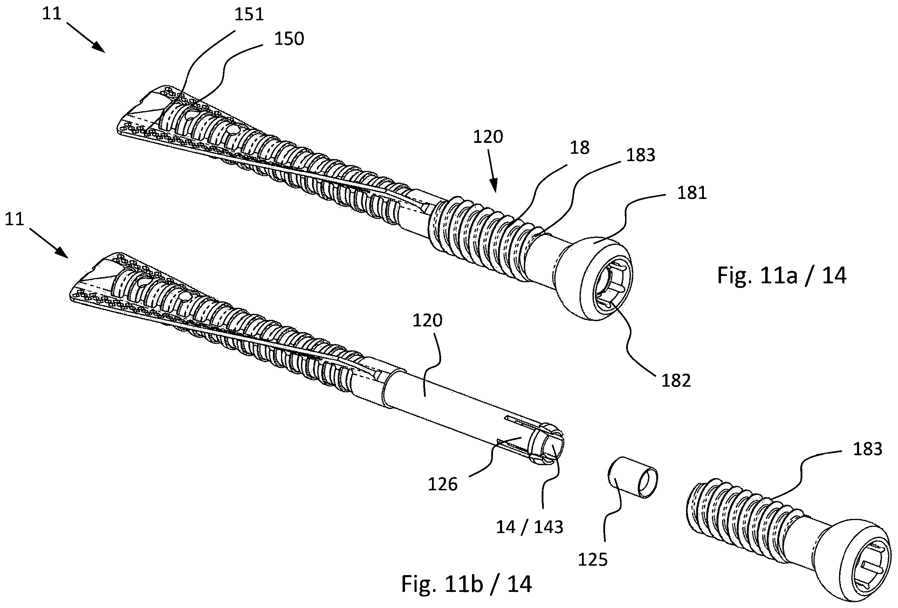

Fig. 11a und 11b zeigen eine alternative Ausführungsform eines Knochenverankerungselements (11). Hierbei ist der Pedikelbereich (120) des Knochenverankerungselements (11) zylindrisch ausgeformt. Darauf gelagert befindet sich ein rotierbarer partieller Gewindeabschnitt (18), welcher ein Knochengewinde (183), einen Kugelkopf (181) und eine Werkzeugeingriffsstelle (182) besitzt. Über das Einleiten einer Drehung auf die Werkzeugeingriffsstelle (182) kann das Knochengewinde aktiviert und so eine Knochenschrauben-ähnliche Verankerung im Pedikelbereich (120) erzielt werden. Somit lassen sich die Vorteile einer Knochenschraube mit einer großflächigen distalen Abstützung verbinden. Gehalten wird der rotierbare partielle Gewindeabschnitt (18) durch elastische Haken (126). Ohne ein Hülsenelement (125) sind die elastischen Haken (126) flexibel. So lässt sich beispielsweise der rotierbare partielle Gewindeabschnitt (18) durch Draufstecken recht einfach montieren. Nach Einführen des Hülsenelements (125) in die Kanülierung (14, 143) sind die elastischen Haken (126) nicht mehr beweglich und es wird somit eine Verliersicherung kreiert.

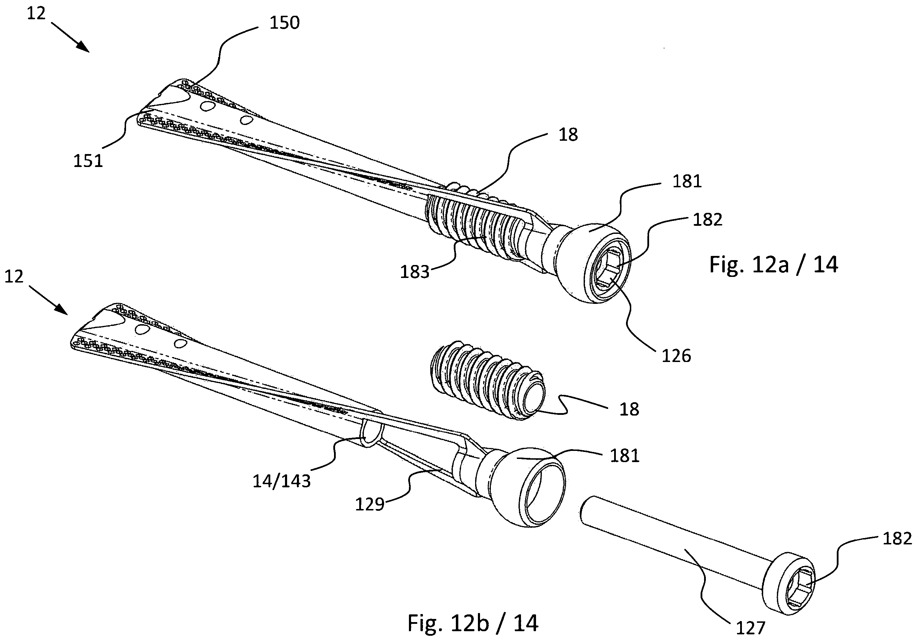

Eine weitere alternative Ausgestaltungsform wird in Fig. 12a und 12b dargestellt. Es wird ein Knochenverankerungselement (12) gezeigt, welches einen rotierbaren partiellen Gewindeabschnitt (18) besitzt. Hierbei befindet sich dieser Gewindeabschnitt (18) in einer dafür vorgesehenen Öffnung (129). Auch hier ist der Gewindeabschnitt (18) drehfest mit einer Werkzeugeingriffsstelle (182) über ein rotierbares Hülsenelement (127) verbunden, allerdings gefügt anstatt einstückig. Der Kugelkopf (181) wird vom Knochenverankerungselement (12) selbst gebildet. Er ist ortsfest im Vergleich zum rotierbaren Hülsenelement (127) angeordnet.

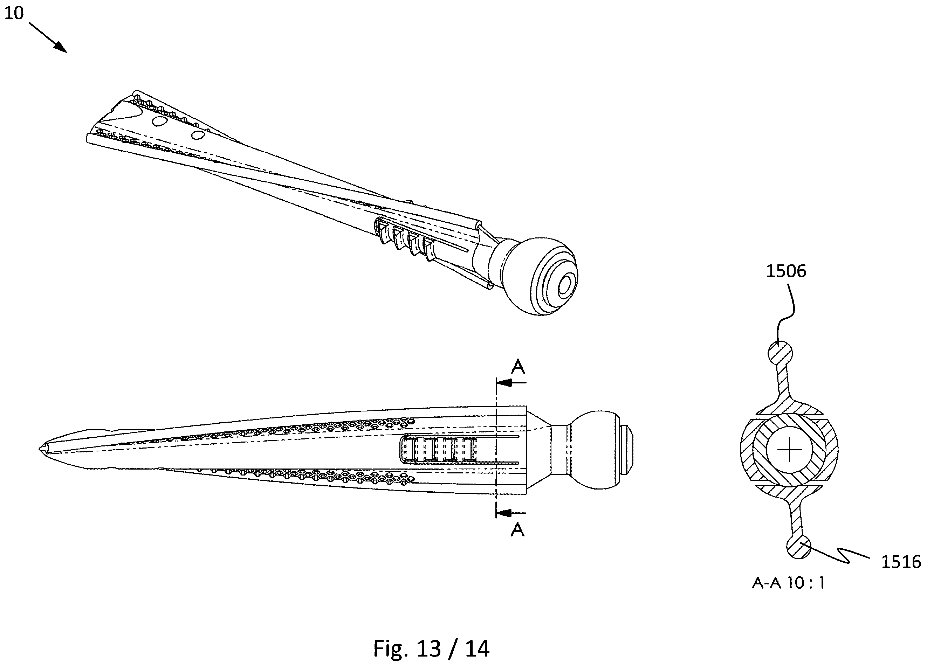

Fig. 13 zeigt eine Form eines Knochenverankerungselements (10) bei dem die Rundungen der Flügelaußenkanten 1506 und 1516 betonter dargestellt sind. Konvex gekrümmte Flächen der Außenkanten haben den Vorteil, dass sie sich nicht in den Knochen einschneiden und Belastungen homogener verteilen. Es kann durchaus vorteilhaft sein, dass die Breite der konvex gekrümmten Flügelaußenkanten größer ist als die Breite der seitlichen Flügelflächen (1502, 1504, 1512 und 1514).

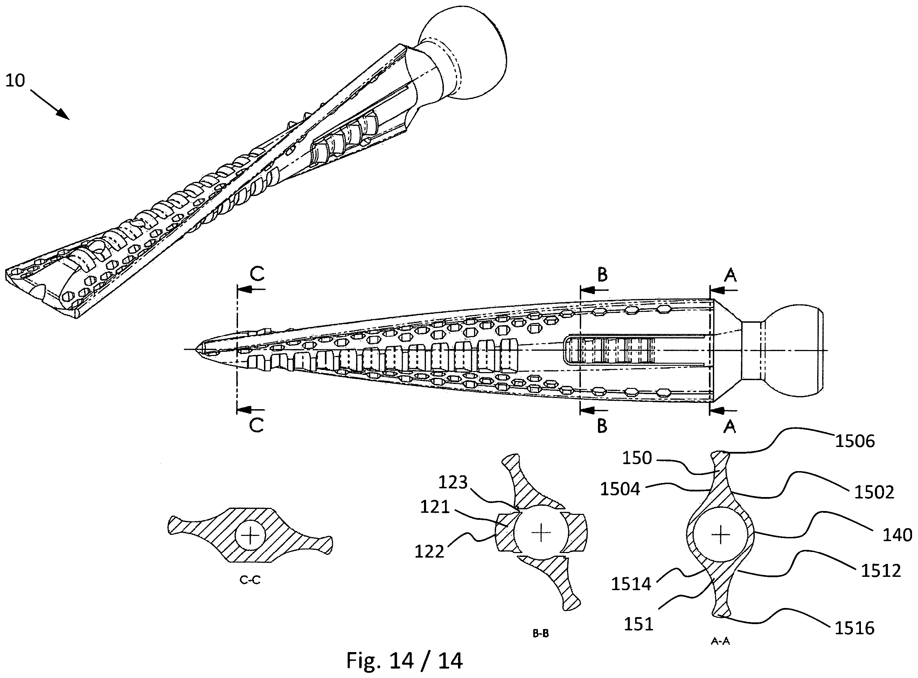

Fig. 14 zeigt eine vorteilhafte Ausführungsform des erfindungsgemäßen Knochenverankerungselements (10) bei dem die Flügel (150 und 151) nicht parallel, sondern im Schnitt zum Kern (140) verdickend zulaufen. Dies hat zur Folge, dass nach radial außen Biegespannungen homogener verteilt werden. Des Weiteren ist auch hier zu erkennen, dass die seitlichen Flächen der Flügel (1502, 1504, 1512 und 1514) einen geringeren Abstand als die konvexen Außenkanten (1506 und 1516) aufweisen können.

## Patentansprüche

1. Knochenverankerungsvorrichtung (1), zum Verankern und Fixieren von Wirbeln (60), insbesondere zum Einführen in einen Pedikelkanal (63), mit einem in einer Seitenansicht u-förmigen Ausschnitt (92) für ein Korrekturelement, insbesondere einen Verbindungsstab (70), aufweisenden Gabelkopf (90) mit zwei Schenkeln (921, 922), die proximal (41) auslaufen und einen Gewindeabschnitt (925) formen, welcher im Eingriff mit einem Stellmittel (80, 81) steht, wobei die Schenkel (921, 922) einen radial äußeren Umfangsbereich aufweisen, in dem zum Ergreifen des Gabelkopfs (90) mittels eines Handhabungsinstruments wenigstens eine Haltenut oder eine sonstige Instrumentenansetzstelle (926) ausgebildet ist,
und einem Knochenverankerungselement (10), mit einem hiervon in axialer Richtung (412) abgewandten proximalen Ende, so dass auch eine distale Richtung (40) und eine proximale Richtung (41) definiert ist, wobei das Knochenverankerungselement (10) am proximalen Bereich einen Kugelkopf (100) besitzt, und das Knochenverankerungselement (10) gegenüber dem Gabelkopf (90) polyaxial verschwenkbar ist, und das Knochenverankerungselement (10) von distal kommend mit dem Gabelkopf (90) montiert ist,
**dadurch gekennzeichnet, dass**
das Knochenverankerungselement (10) einen hauptsächlich zylindrischen Kern (140) besitzt, und der hauptsächlich zylindrische Kern (140) am proximalen Bereich (110, 120), sich wenigstens abschnittsweise (111) von distal (40) nach proximal (41) vergrößert, und zwei Flügel (150, 151) sich seitlich erstrecken, und die Flügel (150, 151) eine distale Flügelausrichtung (440) und eine davon unterschiedliche proximale Flügelausrichtung (441) besitzen, und die Flügel (150, 151) sich helikal zwischen diesen Flügelausrichtungen (440, 441) ausformen und das Knochenverankerungselement (10) nicht zum Einschrauben, sondern zum Einschlagen in den Knochen (61, 63) ausgebildet ist, und das Knochenverankerungselement (10) ferner derart ausgebildet ist, dass nach der Implantation in einen Wirbel am proximalen Bereich die Außenflächen (1506, 1516) der Flügel in Richtung der kranialen und kaudalen Bereiche des Pedikelkanals (63) zeigen und distal (40) die projizierte Fläche (190), die sich durch den Kern (140, 130) und die seitlichen Flügelflächen (1502, 1504, 1512, 1514) ergibt, in der Spongiosa des Wirbels (62) nach kranial (42) /kaudal (43) ausgerichtet ist.

2. Knochenverankerungsvorrichtung gemäß vorherigem Anspruch, **dadurch gekennzeichnet, dass** die Breite der beiden Flügel (150, 151) sich am proximalen Bereich (1510) zum Kugelkopf (100) hin verjüngen und auf der Außenkontur des Kerns (140) münden.

3. Knochenverankerungsvorrichtung gemäß einem der vorherigen Ansprüche, **dadurch gekennzeichnet, dass** das Knochenverankerungselement (10) einen Pedikelbereich (120) besitzt, welcher mindestens eine federelastische Zunge (121) besitzt, welche sich durch einen u-förmigen Schlitz (123) ergibt, und diese mindestens eine federelastische Zunge (121) durch ein einsetzbares Hülsenelement (124, 125) von der federelastischen Beweglichkeit gehemmt wird.

4. Knochenverankerungsvorrichtung gemäß vorherigem Anspruch, **dadurch gekennzeichnet, dass** die federelastische Zunge (121) ein oder mehr Zähne (122) besitzt, und diese Zähne im Pedikelbereich (120) mit dem Pedikelkanal (63) kommunizieren und sich nach Einsetzen des Hülsenelements (124) mit dem Knochen verrasten.

5. Knochenverankerungsvorrichtung gemäß einem der vorherigen Ansprüche, **dadurch gekennzeichnet, dass** das Knochenverankerungselement (10), mit den beiden Flügeln (150, 151) eine Höhe H zwischen den Außenkanten der Flügel (1506 und 1516) definiert, und ein Außendurchmesser D der Zähne (122) definiert, wobei der Formfaktor, aus dem Verhältnis aus H/D, zwischen 1,3 bis 2,5, vorzugsweise 1,4 bis 2,2, vorzugsweise 1,6 bis 2,0 beträgt.

6. Knochenverankerungsvorrichtung gemäß einem der vorherigen Ansprüche, **dadurch gekennzeichnet, dass** das Knochenverankerungselement (10), mindestens eine umlaufende Rille (149) am Kern (140) besitzt, und die umlaufende Rille ein Haken-ähnliches Profil bildet, welches sich in Auszugsrichtung mit dem Knochen verhakt.

7. Knochenverankerungsvorrichtung gemäß einem der vorherigen Ansprüche, **dadurch gekennzeichnet, dass** die Außenflächen (1506, 1516) im Schnitt der Flügel (150 und 151) konvexe Krümmungen besitzen, um die Kontaktspannungen zu den kranialen und kaudalen Pedikelbereichen zu reduzieren.

8. Knochenverankerungsvorrichtung gemäß einem der vorherigen Ansprüche, **dadurch gekennzeichnet, dass** die Außenflächen (1506, 1516) der Flügel (150 und 151), im Schnitt quer zur Achse (412), breiter sind als der minimale Abstand zwischen den Flügelflächen (1502, 1504 und/oder 1512, 1514).

9. Knochenverankerungsvorrichtung gemäß einem der vorherigen Ansprüche, **dadurch gekennzeichnet, dass** die seitlichen Flügelflächen (1502, 1504, 1512, 1514) im Schnitt quer zur Achse (412) sich zum Kern (140) hin verdicken.

10. Knochenverankerungsvorrichtung gemäß einem der vorherigen Ansprüche, **dadurch gekennzeichnet, dass** die Flügel (150 und 151) mehrere Öffnungen (1509, 1519) besitzen, die eine Porengröße zwischen 0,4 und 2,0mm, vorzugsweise 0,5-1,0mm, aufweisen, und diese Öffnungen sechseckig sind und insgesamt dem Anwachsen von Knochen dienen.

11. Knochenverankerungsvorrichtung gemäß einem der vorherigen Ansprüche, **dadurch gekennzeichnet, dass** das Knochenverankerungselement (10) mit Hilfe eines additiven Herstellungsverfahrens hergestellt wurde, wie beispielsweise ein 3d-Druckverfahren, wie das Laserstrahl- oder Elektronstrahl-Schmelzen, und das Knochenverankerungselement (10) einstückig ist,

12. Knochenverankerungsvorrichtung gemäß einem der vorherigen Ansprüche, **dadurch gekennzeichnet, dass** der distale Bereich (130) des Knochenverankerungselements (10) eine Kanülierung (14) und Fenestrationsöffnungen (141) zur Knochenzementaugmentation besitzt, die, wenn das Knochenverankerungselements (10) im Wirbel (60) platziert ist, nach kranial und kaudal gerichtet sind.

13. Knochenverankerungsvorrichtung gemäß einem der vorherigen Ansprüche, **dadurch gekennzeichnet, dass** die Steigung der Flügel (150, 151) zwischen den beiden Flügelausrichtungen (440 und 441) zwischen 100mm bis 300mm beträgt, insbesondere 150mm bis 250mm, insbesondere 160mm bis 200mm.

14. Knochenverankerungsvorrichtung gemäß einem der vorherigen Ansprüche, **dadurch gekennzeichnet, dass** das Knochenverankerungselement (10) derart ausgebildet ist, dass vor dem Einbringen des Knochenverankerungselements (10) die distale Flügelausrichtung (440) der Hauptausrichtung des Pedikels in kranial-kaudaler Richtung (46) entspricht, und in finaler Position die proximale Flügelausrichtung (441) nun der Pedikelhauptausrichtung (46) entspricht, und die distale Flügelausrichtung (440) im Wesentlichen rechtwinklig zur Pedikelhauptausrichtung (46) steht.

15. Knochenverankerungsvorrichtung gemäß einem der vorherigen Ansprüche, **dadurch gekennzeichnet, dass** der Gabelkopf ein Druckstück (91) aufweist, wobei das Druckstück (91) das Knochenverankerungselement (10) am Kugelkopf (100) teilweise umgibt, und das Druckstück (91) am distalen Bereich mindestens einen Schlitz (912) ausformt, das Druckstück (91) so einen federelastischen Bereich (911) besitzt, damit das Druckstück (91) den Kugelkopf (100) federelastisch umschießen kann, und das Druckstück (91) proximal einen Sitz für den Verbindungsstab (913) formt.

16. Knochenverankerungsvorrichtung gemäß einem der Ansprüche 3, 4 oder 7, **dadurch gekennzeichnet, dass** der Pedikelbereich (120) des Knochenverankerungselements (10, 11, 12) einen partiellen Knochengewindeabschnitt (18) besitzt, dieser partielle Knochengewindeabschnitt (18) mit einer Werkzeugansatzstelle (182) gefügt oder verbunden ist, und der Knochengewindeabschnitt (18) drehbar aber ortsfest gegenüber dem Knochenverankerungselement (10, 11, 12) gelagert ist.

17. Knochenverankerungsvorrichtung gemäß einem der vorherigen Ansprüche, **dadurch gekennzeichnet, dass** das Knochenverankerungselement (10) einen Indikator (185) besitzt, welcher Rückschluss auf die distale Flügelausrichtung (440) und/oder proximalen Flügelausrichtung (441) erlaubt.

## Claims

1. A bone anchoring device (1) for anchoring and fixing vertebrae (60), in particular for insertion into a pedicle canal (63), with a fork head (90) having a U-shaped cutout (92) in a side view for a correction element, in particular a connecting rod (70) with two legs (921, 922) which terminate proximally (41) and form a threaded section (925) which engages with an adjusting means (80, 81), wherein the legs (921, 922) have a radially outer circumferential area in which at least one retaining groove or other instrument attachment point (926) is formed for gripping the fork head (90) by means of a handling instrument, and a bone anchoring element (10), with a proximal end facing away therefrom in the axial direction (412), such that a distal direction (40) and a proximal direction (41) are also defined, wherein the bone anchoring element (10) has a spherical head (100) at the proximal end area and the bone anchoring element (10) can be pivoted polyaxially with respect to the fork head (90), and the bone anchoring element (10) is mounted with the fork head (90) coming from the distal end,
**characterized in that**
the bone anchoring element (10) has a mainly cylindrical core (140), and the mainly cylindrical core (140) at the proximal area (110, 120) enlarges at least in sections (111) from distal (40) to proximal (41) and **in that** two wings (150, 151) extend laterally and the wings (150, 151) have a distal wing orientation (440) and a proximal wing orientation (441) different therefrom, and **in that** the wings (150, 151) form helically between these wing orientations (440, 441), and **in that** the bone anchoring element (10) is not formed for screwing in, but for hammering into the bone (61, 63) and **in that**, the bone anchoring element (10) is configured in such a way that after implantation into a vertebra, the outer surfaces (1506, 1516) of the wings at the proximal area point in the direction of the cranial and caudal areas of the pedicle canal (63) and distally (40), the projected area (190) that is formed by the core (140, 130) and the lateral wing surfaces (1502, 1504, 1512, 1514), is aligned in the cranial (42) / caudal (43) direction in the cancellous bone of the vertebra (62).

2. The bone anchoring device according to the preceding claim, **characterized in that** the two wings (150, 151) taper in width at the proximal area (1510) towards the spherical head (100) and open onto the outer contour of the core (140).

3. The bone anchoring device according to any one of the preceding claims, **characterized in that** the bone anchoring element (10) has a pedicle area (120) which has at least one resilient tongue (121) which results from a U-shaped slot (123), and **in that** the resilient mobility of this at least one resilient tongue (121) is inhibited by an insertable sleeve element (124, 125).

4. The bone anchoring device according to the preceding claim, **characterized in that** the resilient tongue (121) has one or more teeth (122), and **in that** these teeth communicate with the pedicle canal (63) in the pedicle area (120) and, after insertion of the sleeve element (124), lock into place with the bone.

5. The bone anchoring device according to any one of the preceding claims, **characterized in that** the bone anchoring element (10) with the two wings (150, 151) defines a height H between the outer edges of the wings (1506 and 1516), and an outer diameter D of the teeth (122), wherein the form factor, from the ratio H/D, is between 1.3 to 2.5, preferably 1.4 to 2.2, preferably 1.6 to 2.0.

6. The bone anchoring device according to any one of the preceding claims, **characterized in that** the bone anchoring element (10) has at least one circumferential groove (149) on the core (140), and **in that** the circumferential groove forms a hook- like profile which hooks with the bone in the pull-out direction.

7. The bone anchoring device according to any one of the preceding claims, **characterized in that** the outer surfaces (1506, 1516) in the section of the wings (150 and 151) have convex curvatures in order to reduce the contact stresses with respect to the cranial and caudal pedicle areas.

8. The bone anchoring device according to any one of the preceding claims, **characterized in that** the outer surfaces (1506, 1516) of the wings (150 and 151), in section transverse to the axis (412), are wider than the minimum distance between the wing surfaces (1502, 1504 and/or 1512, 1514).

9. The bone anchoring device according to any one of the preceding claims, **characterized in that** the lateral wing surfaces (1502, 1504, 1512, 1514) thicken towards the core (140) in section transverse to the axis (412).

10. The bone anchoring device according to any one of the preceding claims, **characterized in that** the wings (150 and 151) have several openings (1509, 1519) which have a pore size between 0.4 and 2.0 mm, preferably 0.5-1.0 mm, and **in that** these openings are hexagonal and are used as a whole for the growth of bones.

11. The bone anchoring device according to any one of the preceding claims, **characterized in that** the bone anchoring element (10) was manufactured with the aid of an additive manufacturing method, such as, for example, a 3D printing process, such as laser beam or electron beam melting, and **in that** the bone anchoring element (10) is in one piece.

12. The bone anchoring device according to any one of the preceding claims, **characterized in that** the distal region (130) of the bone anchoring element (10) has a cannulation (14) and fenestration openings (141) for bone cement augmentation, which are oriented cranially and caudally when the bone anchoring element (10) is placed in the vertebra (60).

13. The bone anchoring device according to any one of the preceding claims, **characterized in that** the pitch of the wings (150, 151) between the two wing orientations (440 and 441) is between 100 mm to 300 mm, in particular 150 mm to 250 mm, in particular 160 mm to 200 mm.

14. The bone anchoring device according to any one of the preceding claims, **characterized in that**, that the bone anchoring element (10) is configured in such a way that before the insertion of the bone anchoring element (10), the distal wing orientation (440) corresponds to the main orientation of the pedicle in the cranial- caudal direction (46), and, in the final position, the proximal wing orientation (441) now corresponds to the main pedicle orientation (46), and **in that** the distal wing orientation (440) is substantially perpendicular to the main pedicle orientation (46).

15. The bone anchoring device according to any one of the preceding claims, **characterized in that** the fork head has a pressure piece (91), wherein the pressure piece (91) partially surrounds the bone anchoring element (10) on the ball head (100), and the pressure piece (91) forms at the distal area at least one slot (912), **in that** the pressure piece (91) has a resilient area (911) such that the pressure piece (91) can enclose the ball head (100) in a resilient manner, and **in that** the pressure piece (91) proximally forms a seat for the connecting rod (913).

16. The bone anchoring device according to any one of the claims 3, 4 or 7, **characterized in that** the pedicle area (120) of the bone anchoring element (10, 11, 12) has a partial threaded bone section (18), **in that** this partial threaded bone section (18) is joined with or connected to a tool attachment point (182), and **in that** the threaded bone section (18) is rotatably but fixedly mounted with respect to the bone anchoring element (10, 11, 12).

17. The bone anchoring device according to any one of the preceding claims, **characterized in that** the bone anchoring element (10) has an indicator (185) which allows inferring the distal wing alignment (440) and/or proximal wing alignment (441).

## Revendications

1. Dispositif d'ancrage osseux (1) pour l'ancrage et la fixation de vertèbres (60), en particulier pour l'insertion dans un canal pédiculaire (63), comportant une tête de fourche (90) ayant une découpe (92) en forme de U dans une vue latérale pour un élément de correction, en particulier une tige de liaison (70), avec deux branches (921, 922) qui s'étendent proximalement (41) et forment une section filetée (925) qui est en prise avec un moyen de réglage (80, 81), les branches (921, 922) présentant une zone périphérique radialement extérieure dans laquelle au moins une rainure de retenue ou un autre point d'attache d'instrument (926) est formée pour saisir la tête de fourche (90) au moyen d'un instrument de manipulation, et un élément d'ancrage osseux (10) avec une extrémité proximale opposée à celle-ci dans la direction axiale (412), de sorte qu'une direction distale (40) et une direction proximale (41) sont également définies, l'élément d'ancrage osseux (10) possèdant une tête de fourche (100) dans la zone proximale et l'élément d'ancrage osseux (10) est pivotant polyaxialement par rapport à la tête de fourche (90) et l"élément d'ancrage osseux (10) est monté à partir de la partie distale avec la tête de fourche (90),
**caractérisé en ce que**
l'élément d'ancrage osseux (10) possède un noyau (140) principalement cylindrique, et le noyau (140) principalement cylindrique sur la zone proximale (110, 120), s'élargit au moins par sections (111) de distal (40) à proximal (41), et deux ailes (150, 151) s'étendent latéralement, et les ailes (150, 151) ont un alignement d'aile distal (440) et un alignement d'aile proximal différent (441), et les ailes (150, 151) se forment hélicoïdalement entre ces alignements d'aile (440, 441) et l'élément d'ancrage osseux (10) est conçu non pas pour être vissé, mais pour être enfoncé dans l'os (61, 63), et l'élément d'ancrage osseux (10) est en outre conçu de telle sorte que, après l'implantation dans une vertèbre sur la zone proximale, les surfaces externes (1506, 1516) des ailes sont alignées dans la direction des zones crânienne et caudale du canal pédiculaire (63) et, au niveau distal (40), la surface projetée (190) qui résulte du noyau (140, 130) et des surfaces latérales des ailes (1502, 1504, 1512, 1514), est orientée dans la spongieuse de la vertèbre (62) vers la direction crânienne (42) / caudale (43).

2. Dispositif d'ancrage osseux selon la revendication précédente, **caractérisé en ce que** la largeur des deux ailes (150, 151) se rétrécit au niveau de la zone proximale (1510) vers la tête de fourche (100) et débouche sur le contour extérieur du noyau (140).

3. Dispositif d'ancrage osseux selon l'une quelconque des revendications précédentes, **caractérisé en ce que** l'élément d'ancrage osseux (10) possède une zone pédiculaire (120) qui possède au moins une languette élastique (121) qui résulte d'une fente en forme de U (123), et cette au moins une languette élastique (121) est inhibée par un élément de manchon insérable (124, 125) de la mobilité élastique à ressort.

4. Dispositif d'ancrage osseux selon la revendication précédente, **caractérisé en ce que** la languette élastique (121) possède une ou plusieurs dents (122), et ces dents communiquent avec le canal pédiculaire (63) dans la zone pédiculaire (120) et s'enclenchent avec l'os après l'insertion de l'élément de manchon (124).

5. Dispositif d'ancrage osseux selon l'une quelconque des revendications précédentes, **caractérisé en ce que** l'élément d'ancrage osseux (10) définit avec les deux ailes (150, 151) une hauteur H entre les bords extérieurs des ailes (1506 et 1516) et un diamètre extérieur D des dents (122), le facteur de forme, à partir du rapport H/D, étant compris entre 1,3 et 2,5, de préférence entre 1,4 et 2,2, de préférence entre 1,6 et 2,0.

6. Dispositif d'ancrage osseux selon l'une quelconque des revendications précédentes, **caractérisé en ce que** l'élément d'ancrage osseux (10) possède au moins une rainure circonférentielle (149) sur le noyau (140), et la rainure circonférentielle forme un profil en forme de crochet qui s'accroche à l'os dans la direction d'extraction.

7. Dispositif d'ancrage osseux selon l'une quelconque des revendications précédentes, **caractérisé en ce que** les surfaces externes (1506, 1516) présentent des courbures convexes dans la section des ailes (150 et 151) afin de réduire les contraintes de contact avec les zones pédiculaires crâniennes et caudales.

8. Dispositif d'ancrage osseux selon l'une quelconque des revendications précédentes, **caractérisé en ce que** les surfaces extérieures (1506, 1516) des ailes (150 et 151), en coupe transversalement à l'axe (412), sont plus larges que la distance minimale entre les surfaces des ailes (1502, 1504 et/ou 1512, 1514).

9. Dispositif d'ancrage osseux selon l'une quelconque des revendications précédentes, **caractérisé en ce que** les surfaces latérales des ailes (1502, 1504, 1512, 1514) s'épaississent en coupe transversalement à l'axe (412) en direction du noyau (140).

10. Dispositif d'ancrage osseux selon l'une quelconque des revendications précédentes, **caractérisé en ce que** les ailes (150 et 151) comportent plusieurs ouvertures (1509, 1519) ayant une taille de pore comprise entre 0,4 et 2,0mm, de préférence 0,5-1,0mm, ces ouvertures étant hexagonales et servant globalement à la croissance des os.

11. Dispositif d'ancrage osseux selon l'une quelconque des revendications précédentes, **caractérisé en ce que** l'élément d'ancrage osseux (10) a été fabriqué à l'aide d'un procédé de fabrication additive, tel qu'un procédé d'impression 3D, tel que la fusion par faisceau laser ou par faisceau électronique, et l'élément d'ancrage osseux (10) est en un seul tenant.

12. Dispositif d'ancrage osseux selon l'une quelconque des revendications précédentes, **caractérisé en ce que** la zone distale (130) de l'élément d'ancrage osseux (10) possède une canulation (14) et des ouvertures de fenestration (141) pour l'augmentation du ciment osseux qui, lorsque l'élément d'ancrage osseux (10) est placé dans la vertèbre (60), sont dirigées dans la direction crânienne et caudale.

13. Dispositif d'ancrage osseux selon l'une quelconque des revendications précédentes, **caractérisé en ce que** le pas des ailes (150, 151) entre les deux alignements d'ailes (440 et 441) est compris entre 100mm et 300mm, notamment entre 150mm et 250mm, notamment entre 160mm et 200mm.

14. Dispositif d'ancrage osseux selon l'une quelconque des revendications précédentes, **caractérisé en ce que** l'élément d'ancrage osseux (10) est conçu de telle sorte que, avant l'insertion de l'élément d'ancrage osseux (10), l'alignement d'aile distale (440) correspond à l'alignement principal du pédicule dans la direction crânio-caudale (46), et en position finale, l'alignement d'aile proximale (441) correspond désormais à l'alignement principal du pédicule (46), et l'alignement d'aile distale (440) est sensiblement perpendiculaire à l'alignement principal du pédicule (46).

15. Dispositif d'ancrage osseux selon l'une quelconque des revendications précédentes, **caractérisé en ce que** la tête de fourche présente une pièce de pression (91), la pièce de pression (91) entourant partiellement l'élément d'ancrage osseux (10) sur la tête de fourche (100), et la pièce de pression (91) formant au moins une fente (912) dans la zone distale, la pièce de pression (91) possédant ainsi une zone élastique (911) de sorte que la pièce de pression (91) puisse entourer élastiquement la tête de fourche (100), et la pièce de pression (91) formant proximalement un siège pour la tige de liaison (913).

16. Dispositif d'ancrage osseux selon l'une quelconque des revendications 3, 4 ou 7, **caractérisé en ce que** la zone pédiculaire (120) de l'élément d'ancrage osseux (10, 11, 12) possède une section de filetage osseux partiel (18), cette section de filetage osseux partiel (18) est assemblée ou reliée à un point d'attache d'outil (182), et la section de filetage osseux (18) est montée de manière rotative mais fixe par rapport à l'élément d'ancrage osseux (10, 11, 12).

17. Dispositif d'ancrage osseux selon l'une quelconque des revendications précédentes, **caractérisé en ce que** l'élément d'ancrage osseux (10) possède un indicateur (185) qui permet de tirer des conclusions sur l'alignement distale des ailes (440) et/ou l'alignement proximale des ailes (441).
